# EUROPEAN PATENT SPECIFICATION

(11) **EP 1 436 331 B1**
(45) Date of publication and mention of the grant of the patent: **20.06.2007**
(21) Application number: 01982703.9
(22) Date of filing: 17.10.2001
(51) Int. Cl.: C07K 16/02, C07K 16/44, C07K 1/36

(54) **PROCESS FOR THE PRODUCTION OF EGG YOLK ANTIBODIES AGAINST ORGANOCHLORINE PESTICIDES**
VERFAHREN ZUR HERSTELLUNG VON ANTIKÖRPERN AUS EIDOTTERN GEGEN ORGANOCHLORINE PESTIZIDEN
PROCEDE RELATIF A L'ELABORATION D'ANTICORPS A BASE DE JAUNE D'OEUF POUR PESTICIDES ORGANOCHLORES

(43) Date of publication of application: 14.07.2004
(73) Proprietor: Council of Scientific and Industrial Research;an Indian Reg. body incorporated under Reg. of Societies Act (Act XXI of 1860), New Delhi 110 001 (IN)
(72) Inventor: RANI, Bangalore Eshwar Amita, 570 013 Karnataka (IN); PASHA, Akmal, 570 013 Karnataka (IN); KARANTH, Nitturu Gopalakrishna, 570 013 Karnataka (IN); RAO, Raja Rao Jagannath, 570 013 Karnataka (IN); GOWDA, Putte, 570 013 Karnataka (IN)
(74) Representative: Fairbairn, Angus Chisholm
(86) International application number: PCT/IN2001/000181
(87) International publication number: WO 2003/033537

(56) References cited:
- WO-A-92/12427
- WO-A-93/21949
- WO-A-96/15249
- DE-A- 19 737 453
- US-A- 4 550 019
- A. ABAD ET AL.: "Hapten synthesis and production of monoclonal antibodies to DDT and related compounds." JOURNAL OF AGRICULTURAL AND FOOD CHEMISTRY, vol. 45, no. 9, 1997, pages 3694-3702, XP002201657

## Description

### TECHNICAL FIELD

The present invention relates to the production of egg yolk antibodies binding to small molecule organochlorine pesticides. The present invention also relates to periodic immunization of the poultry birds with a desired hapten-protein conjugate in the breast muscle.

### BACKGROUND ART

The organochlorine pesticides are large class of insecticides that generally accumulate in the tissues and are difficult to degrade as they have very long half-life periods. Organochlorine insecticides are used widely for treatment of crops such as cereals, fruits, vegetables, legumes, cotton, coffee and tea, forest trees, sanitation and domestic mosquito/malaria control programs.

Small organic molecules such as pesticides are not by themselves immunogenic, but can be made immunogenic following chemical conjugation to a suitable carrier such as a protein. The site and nature of conjugation and carrier protein can influence the specificity and affinity of antibodies produced, in a manner not fully able to be predicted by those skilled in the art. For example, preparation of DDA - GABA (a compound closely related to the hapten used in the present invention coupled with-bovine serum albumin) did not yield conjugates producing antisera with useful reaction with - DDT. (Amitarani, B.E., Akmal Pasha and Karanth N.G.K. (1998, unpublished data).

In recent years, techniques of producing antibodies (polyclonal and monoclonal) have been developed which make it possible to obtain homogeneous, highly specific antibodies. Generally, polyclonal antibodies are used extensively in diagnostics industry. Most commonly they are raised in mammals such as rabbits, mice, rats, horses and goats. This form of antibody production has several disadvantages - large mammals are expensive to maintain, while small mammals yield small quantities of antibody. In addition there is a requirement for periodic drawing of blood from the animals (Deignan et al. 2000)). The amount of IgG (antibody) obtained is usually between 3-8 mg/ ml of serum. The method also involves bleeding of the rabbit several times to obtain the antibodies, as the titer is highest only between the 8^{th} - 10^{th} day after 2 - 3 boosters. Monoclonal antibodies are produced by immunizing an animal with a protein, obtaining antibody-producing cells from the animal, and fusing the antibody-producing cells with strains of myeloma cells, i.e., tumor cells, to produce hybridomas, which are isolated and cultured as monoclones. The monoclonal hybridomas may either be cultured in vitro or from the cells, ascitic fluid, or serum of a tumor-bearing host animal. Since each antibody-producing cell generates a single unique antibody, each monoclonal culture of hybridomas produces a homogeneous antibody. Not all of the hybridoma clones, which result from fusing myeloma cells with antibody-producing cells, are specific for the desired pesticide (or for functional groups upon the pesticide characteristic of that class of molecules), since many of the hybridomas will make antibodies, which the inoculated animal has produced to react with other foreign substances. Even antibodies against the subject antigen will differ from clone to clone, since antibodies produced by different cells may react with different antigenic determinants of the same molecule. From each clone, therefore, it is necessary to obtain the resulting antibody and test its reactivity with the subject pesticides and to test its specificity by determining which particular organochlorine pesticide it recognizes. Further, only certain antibodies or antisera function in specific immunoassay formats or configurations. United States Patent Nos. 4,387,272 and 4,550,019 to Polson; and Losch, U claimed production of hen egg yolk antibodies and has been used in a number of applications for passive transfer of immunity.

United States Patent No. 4,748,018 to Stolle, et al. discloses a method of passive immunization against bacterial infection comprising a preliminary development of tolerance to HEY by repeated oral ingestion of egg yolk, followed by parenteral injection of HEY antibody to a selected bacterial antigen.

United States Patent No. 5,080,895 to Tokoro discloses prevention of E. Coli diarrhoea in newborn piglets by oral administration of anti-bacterial hen egg yolk antibodies.

Reference is made to Hamada, S., Infection and Immunity 59(11): 4161-4167 (1991); and

Otake, S., J. Dental Research 70(3): 162-166 (1991) reproduced the results of Beck in protecting rats against dental caries by means of passive immunization with orally administered hen egg yolk antibodies against S. mutans.

Reference is made to Bartz, C. et al., J. Infectious Disease 142(3): 439-441 (1980) prevented murine rotaviral infection in mice by the oral administration of the water-soluble fraction of the eggs of immunized hens

Reference is made to Yokoyama, H., et al. Infection and Immunity 60(3): 998-1007 (1992) succeeded in passively protecting neonatal piglets from fatal enterotoxigenic E. coli infection by oral administration of a crude yolk immunoglobulin fraction from the eggs of immunized hens.

Reference is made to the animal studies of Yolken, R. et al., Pediatrics 81(2): 291-295 (1988); and Journal Clint. Immunol. 10(6): 80S-87S (1990), proposed the oral administration of antiviral HEY immunoglobulin for the prevention and treatment of enteric infections, including rotaviral infection in humans. Methods and formulations for the oral administration of immune globulin are known (U.S. Pat. No. 4,477,432 to Hardie). However, there are no reports on the production of egg yolk antibodies for a small molecule such as a pesticide.

The main object of the present invention, is to provide antibodies from the egg yolk of hyper immunized hens (HEY antibody) for organochlorine insecticides.

Another object of the present invention is to provide continuous supply of large quantities of consistent, high titer and specific antibodies that can be easily collected and stored.

Yet another object of the present invention is to provide a non-invasive process, hence no need to bleed the animal.

Still another object of the present invention, is to provide antibodies that are equally or more sensitive to both polyclonal and monoclonal antibodies produced using mammals.

The present invention relates to a process to produce egg yolk antibodies binding to small molecule organochlorine pesticides, by harvesting antibodies from poultry birds previously immunized with a desired hapten-protein conjugate in the breast muscle. Antibodies are harvested from egg yolk after five weeks interval.

Accordingly, the present invention provides a process for the production of egg yolk antibodies binding to small molecule organochlorine pesticides, the said process comprising harvesting antibodies from the egg yolk of immunized poultry birds, wherein said birds have previously been obtained by the steps of :
(a) selecting suitable poultry birds;
(b) immunizing the poultry birds with known complete adjuvant, each ml of said adjuvant comprising heat killed and dried 1 mg of Mycobacterium tuberculosis (H37Ra, ATCC 25177), 0.85 ml paraffin and 0.15 ml mannide monooleate;
(c) immunizing the birds with 1000 µg conjugate selected from DDT-OH hapten, Octachloro cyclic hapten and 2,4,5 trichlorophenoxyacetic acid β-alanine in breast muscle;
(d) immunizing the birds again with the hapten-protein conjugate as given in step (c) with 500 µg of desired hapten conjugate;
(e) immunizing the birds with hapten-protein conjugate at the intervals of two, three and five weeks; and
(f) immunizing the birds thereafter with hapten-protein conjugate at five weeks intervals as long as the bird lays eggs.

In an embodiment of the present invention, the desired hapten-protein conjugates have binding properties to DDT, Endosulphan and HCH.

In another embodiment of the present invention, the hapten-protein conjugate DDT-OH binding to DDT is used and is produced as follows:
(a) succinylating 2,2,-Bis(4-chlorophenyl) -1,1,1-trichloroethanol overnight, using excess succinic anhydride in pyridine to obtain N-hydroxy succunimide;
(b) reacting N-hydroxy succinimide 183.5 mg., 0.5mmol in dichloromethane in the presence of dicyclohexylurea and dimethylaminopyridine catalyst in the ratio 1:1:1:1.2 (hapten:NHS:DCC:DMAP) to convert into N-Hydroxy succinimide active ester, and
(c) obtaining active ester of DDT-OH hapten for use in conjugation by isolating dicyclohexylurea and evaporating dichloromethane.

In yet another embodiment of the present invention, the hapten-protein conjugate octachloro cyclic hapten binding to Endosulphan is used and is produced as follows:
(a) dissolving about 3.73 g Heptachlor in 0.1 mol glacial acetic acid by warming;
(b) dissolving 1.085 g Tert-Butyl hypochlorite, in 0.1 mmol glacial acetic acid and adding to the first solution as obtained in step(a);
(c) refluxing the mixture on a water-bath for 1 hour,
(d) separating fine crystals of acetyl-chloro derivative of heptachlor,
(e) washing the crystals with acetone and drying with air,
(f) obtaining the crystalline product in a yield of about 3.02 g, m.p. 238 C. 1.09 g
(g) treating the product to get the pre-hapten 1,3,4,5,6,7,8,8-Octachloro-2-hydroxy-4, 7-methano-3a, and 4,7,7a-tetrahydroindane;
(h) dissolving the pre hapten in dichloromethane by adding N-hydroxysuccinimide and cooling the mixture to 0°C ;
(i) adding dicyclohexylcarbodiimide followed by dimethylaminopyridine;
(j) stirring the mixture overnight; and
(k) filtering off dichloromethane and evaporating dichloromethane to obtain the active ester of endosulphan.

In still another embodiment of the present invention, the conjugate 2,4,5-Trichloro phenoxy acetic acid β- alanine Trichloro benzene (TCB) hapten binding to Hexachloro hexane is used and is produced as follows:
(a) adding of β - alanine spacer arm to 2,4,5 Trichlorophenoxyacetic acid by suspending 10mM, 2.55g of 2,4,5 Trichlorophenoxyacetic acid in 5.95 ml thionyl chloride (9 50 mmol);
(b) refluxing for 1 hour and removing unreacted thionyl chloride by evaporation;
(c) stirring the product with β - alanine 9 mmol, 0.66g in 7.4 ml of 1M NaOH at 0°C;
(d) warming the product for over 16 hours at room temperature;
(e) isolating the resulting acid by acidification;
(f) partitioning into ethyl acetate;
(g) washing with water and brine;
(h) giving an yield of crude product hapten containing 2,4,5-Trichlorophenoxyacetic acid (2,4,5-T) as impurity;
(i) dissolving the impurity in acetone to obtain colorless flakes of the Trichlorobenzene(TCB) hapten;
(j) filtering and washing the colourless TCB hapten with acetone and drying in air,
(k) using silica gel precoated aluminum plates and a mixture of chloroform and methanol 85:15 as eluent showed a single spot in TLC analysis Rf- 0.45 detected by spraying with 2% o-tolidine in acetone and exposure to Uv light/sunlight, at a melting range of 169-70 °C.
(l) synthesizing the active ester of hapten 2,4,5-T-β- alanine at melting range of 102-104 °C by dissolving in dichloromethane.
(m) adding N- hydroxysuccinimide and the mixture is cooled in an ice-bath;
(n) adding Dimethylsulphoxide(DMSO) dropwise to the mixture until the hapten is dissolved;
(o) adding Dicyclohexylcarbodiimide to the mixture followed by adding dimethylaminopyridine catalyst;
(p) stirring the mixture overnight and the temperature slowly raised to the room temperature;
(q) filtering and evaporating acetone; and
(r) separating the active ester as a colorless solid.

In yet another embodiment of the present invention, harvesting of antibodies is conducted as follows:
(a) obtaining egg yolk without rupturing the yolk;
(b) adding 100 ml of Tris buffer saline for every 10 ml of yolk;
(c) removing the precipitate by centrifugation;
(d) adding to the supernatant the precipitating solution of magnesium chloride and phosphotungstic acid for centrifuging;
(e) discarding the pellet;
(f) adding to the supernatant a water soluble protein fraction 12% polyethylene glycol;
(g) incubating for 10 minutes and then centrifuging again;
(h) precipitating out the antibody;
(i) adding 10 ml of 10mM phosphate buffer to dissolve the precipitate;
(j) cooling the antibody solution 0°Cl;
(k) adding 10 ml of pre-cooled ethanol;
(l) centrifuging the solution at 4°C and dissolving the sediment in 10 mM phosphate buffer, and
(m)dialyzing against phosphate buffer for 24 h at 4°C to obtain the yield of antibodies.

In still another embodiment of the present invention, harvesting of antibodies is conducted as follows:
(a) obtaining the egg yolk from the eggshell without rupturing the yolk membrane;
(b) adding for every 10 ml of yolk, 10 ml of distilled water,
(c) adding about 0.15 % of kappa- carragenanin and left to stir for 30 minutes at room temperature;
(d) filtering and centrifuging the solution at for 15 minutes;
(e) passing through the DEAE - sephacel column prepared with 20 mM phosphate buffer pH 8.0;
(f) eluting with 0.2 M phosphate buffer pH 8.0;
(g) collecting the eluate and the absorbance read at 280 nm; and
(h) pooling and storing the peak fractions containing the antibody at 4°C.

In yet another embodiment of the present invention, the lipid from egg yolk is precipitated out twice using the precipitating solution of phosphotungstic acid and magnesium chloride and centrifuged obtaining the antibody yield up to 75% from supernatant.

In still another embodiment of the present invention, pH of the water soluble protein fraction obtained after the removal of the lipids is adjusted to pH 5.0 to further precipitate out the antibodies for obtaining a yield of 80 -90%.

In yet another embodiment of the present invention, the yield of antibody is to the extent of 73%.

In still another embodiment of the present invention, the hyper immune eggs are collected daily and stored 40°C until further use.

In yet another embodiment of the present invention, the production of the antibody is commenced from 7^{th} day after the immunization and continued for 60 days.

In still another embodiment of the present invention, the titer of the antibody produced is 165-225 mg/ml.

In yet another embodiment of the present invention, the egg yolk antibody produced is more/ equally sensitive to the polyclonal /monoclonal antibodies produced using mammals.

In still another embodiment of the present invention, production of the egg yolk antibodies relates to small molecules of pesticides.

In yet another embodiment of the present invention, the egg yolk antibodies produced bind to the following organochlorine insecticides selected from:
a) (**DDT**-1,1'- (2,2,2 - Trichloroethylene) bis (4 - chlorobenzene),
b) **HCH**-1,2,3,4,5,6-hexachlorohexane
c) **ENDOSULPHAN** - 6,7,8,9,10,10-hexachloro-1,5, 5a,6,9,9a, hexahydro - 6,9-methano-2,3,4- benzodioxathiepin - 3 - oxide.)

The following examples are given by way of illustration of the present invention and therefore should not be construed to limit the scope of the present invention.

### EXAMPLES

### EXAMPLE-1

The egg yolk was carefully removed from the eggshell without rupturing the yolk membrane. The entire albumin adhering to the membrane was washed off. And the egg yolk membrane was ruptured and transferred into a measuring cylinder through a funnel. For every 10 ml of yolk, 100ml of Tris buffer saline was added. The precipitate formed was removed by centrifugation. To the supernatant the precipitating solution of magnesium chloride and phosphotungstic acid was added and centrifuged again. The pellet discarded and to the supernatant now called the water soluble protein fraction 12% polyethylene glycol was added and incubated for 10 minutes and then centrifuged: The antibody precipitates out. 10 ml of 10mM phosphate buffer is added and the precipitate dissolved.

The antibody solution is then cooled to 0°C and 10 ml of pre cooled ethanol added. The solution is centrifuged at 4°C and the sediment is dissolved in 10-mM phosphate buffer and dialyzed in phosphate buffer for 24 h at 4°C. Yield - 60%.

### EXAMPLE -2

The lipid from the egg yolk was precipitated out twice using the precipitating solution of phosphotungstic acid and magnesium chloride from the supernatant and centrifuged.
Yield - 75%.

### EXAMPLE -3

The pH of the water soluble protein fraction obtained after the removal of the lipids was checked and adjusted to pH 5.0 in order to further precipitate out the antibodies.
Yield - 80 - 90%.

### EXAMPLE - 4

The egg yolk was carefully removed from the eggshell without rupturing the yolk membrane. The entire albumin adhering to the membrane was washed off. And the egg yolk membrane was ruptured and transferred into a measuring cylinder through a funnel. For every 10 ml of yolk, 10 ml of distilled water was added. 0.15 % of kappa-carragenanin was added and left to stir for 30 minutes at room temperature. The solution was then filtered and centrifuged at 10, 000g for 15 minutes. The pH of the supernatant solution was set to pH 8.0 and then passed through the DEAE - sephacel column prepared with 20 mM phosphate buffer pH 8.0. The antibody was eluted with 0.2 M phosphate buffer pH 8.0. The eluate was collected and the absorbency read at 280 nm. The peak fractions containing the antibody were pooled and stored at 4°C. Yield - 73 %.

The efficacy of these antibodies has also been inferred through this invention.

### ADVANTAGES

The main advantages of the present invention are:
(a) The use of antibodies from the egg yolks of hyperimmunized hens (HEY antibody) for immunological procedures overcome the limitations associated with the polyclonal and monoclonal antibodies, because the present method provides a continuous supply of large quantities of consistent, high titer specific and sensitive antibody which can be easily collected and stored.
(b) The sensitivity of the assay is equal or better than rabbit antibodies.
(c) The method of harvesting/sampling the antibodies is non-invasive and the produced antibodies have a good affinity to the analyte.

The advantages of antibodies produced by chicken over rabbit are further illustrated in the tabular form as provided in the following Table I.

**TABLE I**

| **PARAMETERS** | **LAYING HENS(4 NOS)** | **RABBITS(2-4 NOS)** |
|---|---|---|
| **A. MAINTENANCE COST IN Rs.** | | |
| **(a) Investment for cage** | **15,000** | **20,000** |
| **(ii) Price for each animal** | **60** | **75** |

| **B.ANTIBODY** | **40 mg/ml** | **7mg/ml** |
|---|---|---|
| **PRODUCTION** | **2000-2800mg** | **200 mg** |
| **(i) Monthly** | | |
| **(ii)Amount of specific antibody** | **upto 10%** | **5%** |

| **C. INFRASTRUCTURE** | **Poultry birds** | **Small animals** |
|---|---|---|
| **Area needed** | | |

| **D. CHARACTERISTICS** | | |
|---|---|---|
| **i) Antibody sampling** | **Non-invasive** | **Invasive** |
| **ii) Purification** | **Affinity** | **Affinity** |
| **iii) Interference with rheumatoid factor** | **No** | **Yes** |
| **iv) Activation of mammalian complement** | **No** | **Yes** |

## Claims

1. A process for the production of egg yolk antibodies binding to small molecule organo chlorine pesticides, the said process comprising harvesting antibodies from the egg yolk of immunized poultry birds, wherein said birds have previously been obtained by the steps of:
(a) selecting suitable poultry birds;
(b) immunizing the poultry birds with known complete adjuvant, each ml of said adjuvant comprising heat killed and dried 1 mg of Mycobacterium tuberculosis (H37Ra, ATCC 25177), 0.85 ml paraffin and 0.15 ml mannide monooleate;
(c) immunizing the birds with 1000 µg conjugate selected from DDT-OH hapten, Octachloro cyclic hapten, 2,4,5 trichlorophenoxyacetic acid β-alanine in breast muscle;
(d) immunizing the birds again with the hapten-protein conjugate as given in step (c) with 500 µg of desired hapten conjugate;
(e) immunizing the birds with hapten-protein conjugate at the intervals of two, three and five weeks; and
(f) immunizing the birds thereafter with hapten-protein conjugate at five weeks intervals as long as the bird lays eggs.

2. A process as claimed in claim 1, wherein the desired hapten-protein conjugates have binding properties to DDT, Endosulphan and Hexachlorohexane.

3. A process as claimed in claim 1, wherein the hapten-protein conjugate DDT-OH binding to DDT is used in step (c) of claim 1, and is produced as follows:
(a) succinylating 2,2,-Bis(4-chlorophenyl) -1,1,1-trichloroethanol overnight, using excess succinic anhydride in pyridine to obtain N-hydroxy succunimide;
(b) reacting N-hydroxy succinimide 183.5 mg., 0.5mmol in dichloromethane in the presence of dicyclohexylurea and dimethylaminopyridine catalyst in the ratio 1:1:1:1.2 (hapten:NHS:DCC:DMAP) to convert into N-Hydroxy succinimide active ester, and
(c) obtaining active ester of DDT-OH hapten for use in conjugation by isolating dicyclohexylurea and evaporating dichloromethane.

4. A process as claimed in claim 1, wherein the hapten-protein conjugate octachloro cyclic hapten binding to Endosulphan is used in step(c) of claim 1, and is produced as follows:
(a) dissolving about 3.73 g Heptachlor in 0.1 mol glacial acetic acid by warming;
(b) dissolving 1.085 g Tert-Butyl hypochlorite, in 0.1 mmol glacial acetic acid and adding to the first solution as obtained in step(a);
(c) refluxing the mixture on a water-bath for 1 hour,
(d) separating fine crystals of acetyl-chloro derivative of heptachlor-,
(e) washing the crystals with acetone and drying with air;
(f) obtaining the crystalline product in a yield of about 3.02 g, m.p. 238 C. 1.09 g
(g) treating the product to get the pre-hapten 1,3,4,5,6,7,8,8-Octachloro-2-hydroxy-4, 7-methano-3a, and 4,7,7a-tetrahydroindane;
(h) dissolving the pre hapten in dichloromethane by adding N-hydroxysuccinimide and cooling the mixture to 0°C ;
(i) adding dicyclohexylcarbodiimide followed by dimethylaminopyridine;
(j) stirring the mixture overnight; and
(k) filtering off dichloromethane and evaporating dichloromethane to obtain the active ester of endosulphan.

5. A process as claimed in claim 1, wherein the conjugate hapten 2,4,5-Trichloro phenoxy acetic acid β- alanine (TCB) hapten binding to Hexachloro hexane, is used in step (c) of claim 1, and is produced as follows:
(a) adding of ß - alanine spacer arm to 2,4,5 Trichlorophenoxyacetic acid by suspending 10mM, 2.55g of 2,4,5 Trichlorophenoxyacetic acid in 5.95 ml thionyl chloride (9 50 mmol);
(b) refluxing for 1 hour and removing unreacted thionyl chloride by evaporation;
(c) stirring the product with ß - alanine (9 mmol, 0.66g in 7.4 ml of 1M NaOH) at 0°C;
(d) warming the product for over 16 hours at room temperature;
(e) isolating the resulting acid by acidification;
(f) partitioning into ethyl acetate;
(g) washing with water and brine;
(h) giving an yield of crude product hapten containing 2,4,5-Trichlorophenoxyacetic acid (2,4,5-T) as impurity;
(i) dissolving the impurity in acetone to obtain colorless flakes of the Trichlorobenzene (TCB) hapten;
(j) filtering and washing the colourless TCB hapten with acetone and drying in air,
(k) using silica gel precoated aluminum plates and a mixture of chloroform and methanol in a ratio of 85:15 as eluent showed a single spot in TLC analysis Rf-0.45 detected by spraying with 2% o-tolidine in acetone and exposure to Uᵥ light/sunlight, at a melting range of 169-70 °C.
(l) synthesizing the active ester of hapten 2,4,5-T-β- alanine at melting range of 102-104 °C by dissolving in dichloromethane .
(m) adding N- hydroxysuccinimide and the mixture is cooled in an ice-bath;
(n) adding Dimethylsulphoxide(DMSO) dropwise to the mixture until the hapten is dissolved;
(o) adding Dicyclohexylcarbodiimide to the mixture followed by adding dimethylaminopyridine catalyst;
(p) stirring the mixture overnight and the temperature slowly raised to the room temperature;
(q) filtering and evaporating acetone; and
(r) separating the active ester as a colorless solid.

6. A process as claimed in claim 1, wherein harvesting of antibodies is as follows:
(a) obtaining egg yolk without rupturing the yolk;
(b) adding 100 ml of Tris buffer saline for every 10 ml of yolk;
(c) removing the precipitate by centrifugation;
(d) adding to the supernatant the precipitating solution of magnesium chloride and phosphotungstic acid for centrifuging;
(e) discarding the pellet;
(f) adding to the supernatant a water soluble protein fraction 12% polyethylene glycol;
(g) incubating for 10 minutes and then centrifuging again;
(h) precipitating out the antibody;
(i) adding 10 ml of 10mM phosphate buffer to dissolve the precipitate;
(j) cooling the antibody solution 0°Cl;
(k) adding 10 ml of pre-cooled ethanol;
(l) centrifuging the solution at 4°C and dissolving the sediment in 10 mM phosphate buffer; and
(m)dialyzing against phosphate buffer for 24 h at 4°C to obtain the yield of antibodies.

7. A process as claimed in claim 1, wherein harvesting of antibodies is conducted as follows:
(a) obtaining the egg yolk from the eggshell without rupturing the yolk membrane;
(b) adding for every 10 ml of yolk, 10 ml of distilled water;
(c) adding about 0.15 % of kappa- carragenanin and left to stir for 30 minutes at room temperature;
(d) filtering and centrifuging the solution at for 15 minutes;
(e) passing through the DEAE - sephacel column prepared with 20 mM phosphate buffer pH 8.0;
(f) eluting with 0.2 M phosphate buffer pH 8.0;
(g) collecting the eluate and the absorbance read at 280 nm; and
(h) pooling and storing the peak fractions containing the antibody at 4 ° C.

8. A process as claimed in claim 6, wherein the lipid from egg yolk is precipitated out twice using the precipitating solution of phosphotungstic acid and magnesium chloride and centrifuged obtaining the antibody yield up to 75% from supernatant.

9. A process as claimed in claim 6, wherein pH of the water soluble protein fraction obtained after the removal of the lipids is adjusted to pH 5.0 to further precipitate out the antibodies for obtaining a yield of 80 -90%.

10. A process as claimed in claim 7, wherein the yield of antibody is to the extent of 73%.

11. A process as claimed in claim 1, wherein the hyper immune eggs are collected daily and stored 40°C until further use.

12. A process as claimed in claim 1, wherein the production of the antibody is commenced from 7^{th} day after the immunization and continued for 60 days.

13. A process as claimed in claim 1, wherein the titer of the antibody produced is 165-225 mg/ml.

14. A process as claimed in claim 1, wherein the egg yolk antibody produced is more/equally sensitive to the polyclonal / monoclonal antibodies produced using mammals.

## Patentansprüche

1. Verfahren zur Herstellung von Antikörpern aus Eidottern, bindend an Organochlorpestizide mit kleinen Molekülen, wobei das Verfahren
Ernten von Antikörpern aus dem Eidotter von immunisierten Nutzvögeln umfaßt, wobei die Vögel vorher durch die Schritte:
(a) Auswählen geeigneter Nutzvögel;
(b) Immunisieren der Vögel mit bekanntem kompletten Adjuvans, wobei jeder ml des Adjuvans 1 mg von hitzegetötetem und getrocknetem Mycobacterium tuberculosis (H37Ra, ATCC 25177), 0,85 ml Paraffin und 0,15 ml Mannidmonooleat umfaßt;
(c) Immunisieren der Vögel mit 1000 µg Konjugat, ausgewählt aus DDT-OH-Hapten, cyclischem Octachlor-Hapten, 2,4,5-Trichlorphenoxyessigsäure-β-Alanin, im Brustmuskel;
(d) Immunisieren der Vögel mit wieder dem Hapten-Protein-Konjugat, wie angegeben in Schritt (c), mit 500 µg des gewünschten Hapten-Konjugats;
(e) Immunisieren der Vögel mit Hapten-Protein-Konjugat in den Intervallen von zwei, drei und fünf Wochen; und
(f) Immunisieren der Vögel danach mit Hapten-Protein-Konjugat in fünf-Wochen-Intervallen, so lange, wie der Vogel Eier legt, erhalten worden sind.

2. Verfahren nach Anspruch 1, wobei die gewünschten Hapten-Protein-Konjugate Bindungseigenschaften an DDT, Endosulfan und Hexachlorhexan haben.

3. Verfahren nach Anspruch 1, wobei das Hapten-Protein-Konjugat DDT-OH, bindend an DDT, in Schritt (c) von Anspruch 1 verwendet wird und wie folgt hergestellt wird:
(a) Succinylieren von 2,2-Bis(4-chlorphenyl)-1,1,1-trichlorethanol über Nacht, wobei überschüssiges Succinsäureanhydrid in Pyridin verwendet wird, um N-Hydroxysuccinimid zu erhalten;
(b) Umsetzen von N-Hydroxysuccinimid, 183,5 mg, 0,5 mmol, in Dichlormethan in Anwesenheit von Dicyclohexylharnstoff und Dimethylaminopyridin-Katalysator im Verhältnis 1:1:1:1,2 (Hapten:NHS:DCC:DMAP), um in N-Hydroxysuccinimid-aktiven Ester umzuwandeln; und
(c) Erhalten des aktiven Esters von DDT-OH-Hapten zur Verwendung in Konjugation durch Isolieren von Dicyclohexylharnstoff und Verdampfen von Dichlormethan.

4. Verfahren nach Anspruch 1, wobei das Hapten-Protein-Konjugat cyclisches Octachlor-Hapten, bindend an Endosulfan, in Schritt (c) von Anspruch 1 verwendet wird und wie folgt hergestellt wird:
(a) Lösen von etwa 3,73 g Heptachlor in 0,1 mol Eisessig durch Erwärmen;
(b) Lösen von 1,085 g tert-Butylhypochlorit in 0,1 mmol Eisessig und Hinzufügen zu der ersten Lösung, wie erhalten in Schritt (a);
(c) Erhitzen des Gemisches unter Rückfluß in einem Wasserbad für 1 Stunde;
(d) Abtrennen feiner Kristalle des Acetylchlorderivats von Heptachlor;
(e) Waschen der Kristalle mit Aceton und Trocknen mit Luft;
(f) Erhalten des kristallinen Produkts in einer Ausbeute von etwa 3,02 g, Smp. 238°C, 1,09 g;
(g) Behandeln des Produkts, um das Prähapten 1,3,4,5,6,7,8,8-Octachlor-2-hydroxy-4,7-methano-3a- und -4,7,7a-tetrahydroindan zu erhalten;
(h) Lösen des Prähaptens in Dichlormethan durch Hinzufügen von N-Hydroxysuccinimid und Kühlen des Gemisches auf 0°C;
(i) Hinzufügen von Dicyclohexylcarbodiimid und nachfolgend Dimethylaminopyridin;
(j) Rühren des Gemisches über Nacht; und
(k) Abfiltrieren von Dichlormethan und Verdampfen von Dichlormethan, um den aktiven Ester von Endosulfan zu erhalten.

5. Verfahren nach Anspruch 1, wobei das Konjugat Hapten-2,4,5-Trichlorphenoxyessigsäure-β-Alanin-(TCB)-Hapten, bindend an Hexachlorhexan, in Schritt (c) von Anspruch 1 verwendet wird und wie folgt hergestellt wird:
(a) Hinzufügen von β-Alanin-Spacer-Arm zu 2,4,5-Trichlorphenoxyessigsäure durch Suspendieren von 10 mM, 2,55 g, 2,4,5-Trichlorphenoxyessigsäure in 5,95 ml Thionylchlorid (9 50 mmol);
(b) Erhitzen unter Rückfluß für 1 Stunde und Entfernen von unumgesetztem Thionylchlorid durch Verdampfung;
(c) Rühren des Produkts mit β-Alanin (9 mmol, 0,66 g in 7,4 ml 1M NaOH) bei 0°C;
(d) Erwärmen des Produkts für über 16 Stunden auf Raumtemperatur;
(e) Isolieren der resultierenden Säure durch Ansäuerung;
(f) Partitionieren in Ethylacetat;
(g) Waschen mit Wasser und Kochsalzlösung;
(h) Ergeben einer Ausbeute von Rohprodukt-Hapten, enthaltend 2,4,5-Trichlorphenoxyessigsäure (2,4,5-T) als Verunreinigung;
(i) Lösen der Verunreinigung in Aceton, um farblose Flocken des Trichlorbenzol-(TCB)-Haptens zu erhalten;
(j) Filtrieren und Waschen des farblosen TCB-Haptens mit Aceton und Trocknen in Luft;
(k) Verwenden von Silicagel-vorbeschichteten Aluminiumplatten und einem Gemisch von Chloroform und Methanol in einem Verhältnis von 85:15 als Eluent zeigte einen einzelnen Fleck in der TEC-Analyse Rf 0,45, nachgewiesen durch Sprühen mit 2% o-Tolidin in Aceton und Einwirkung von UV-Licht/Sonnenlicht, mit einem Schmelzbereich von 169-70°C;
(l) Synthetisieren des aktiven Esters von Hapten-2,4,5-T-β-Alanin mit einem Schmelzbereich von 102-104°C durch Lösen in Dichlormethan;
(m) Hinzufügen von N-Hydroxysuccinimid und das Gemisch wird in einem Eisbad gekühlt;
(n) tropfenweises Hinzufügen von Dimethylsulfoxid (DMSO) zu dem Gemisch, bis das Hapten gelöst ist;
(o) Hinzufügen von Dicyclohexylcarbodiimid zu dem Gemisch und nachfolgendes Hinzufügen von Dimethylaminopyridin-Katalysator;
(p) Rühren des Gemisches über Nacht und wobei die Temperatur langsam auf die Raumtemperatur erhöht wird;
(q) Filtrieren und Verdampfen von Aceton; und
(r) Abtrennen des aktiven Esters als farblosen Feststoff.

6. Verfahren nach Anspruch 1, wobei die Antikörper wie folgt geerntet werden:
(a) Erhalten von Eidotter ohne Zerreißen des Dotters;
(b) Hinzufügen von 100 ml Tris-Puffer-Kochsalzlösung für jede 10 ml von Dotter;
(c) Entfernen des Niederschlags durch Zentrifugation;
(d) Hinzufügen der ausfällenden Lösung von Magnesiumchlorid und Phosphorwolframsäure zu dem Überstand zum Zentrifugieren;
(e) Ablegen des Pellets;
(f) Hinzufügen einer wasserlöslichen Proteinfraktion mit 12% Polyethylenglycol zu dem Überstand;
(g) Inkubieren für 10 Minuten und dann wieder Zentrifugieren;
(h) Ausfällen des Antikörpers;
(i) Hinzufügen von 10 ml 10 mM Phosphatpuffer, um den Niederschlag zu lösen;
(j) Kühlen der Antikörperlösung auf 0°C;
(k) Hinzufügen von 10 ml vorgekühltem Ethanol;
(l) Zentrifugieren der Lösung bei 4°C und Lösen des Sediments in 10 mM Phosphatpuffer; und
(m) Dialysieren gegen Phosphatpuffer für 24 h bei 4°C, um die Ausbeute von Antikörpern zu erhalten.

7. Verfahren nach Anspruch 1, wobei das Ernten von Antikörpern wie folgt durchgeführt wird:
(a) Erhalten des Eidotters aus der Eierschale ohne Zerreißen der Dottermembran;
(b) Hinzufügen von 10 ml destilliertem Wasser für jeweils 10 ml Dotter;
(c) Hinzufügen von etwa 0,15% kappa-Carrageenan und Rührenlassen für 30 Minuten bei Raumtemperatur;
(d) Filtrieren und Zentrifugieren der Lösung für 15 Minuten;
(e) Hindurchführen durch die DEAE-Sephacel-Säule, vorbereitet mit 20 mM Phosphatpuffer pH 8,0;
(f) Eluieren mit 0,2 M Phosphatpuffer pH 8,0;
(g) Sammeln des Eluats und der Extinktion die bei 280 nm abgelesen wird; und
(h) Poolen und Lagern der Peakfraktionen, enthaltend den Antikörper, bei 4°C.

8. Verfahren nach Anspruch 6, wobei das Lipid aus Eidotter zweimal unter Verwendung der ausfällenden Lösung von Phosphorwolframsäure und Magnesiumchlorid ausgefällt und zentrifugiert wird, wobei aus dem Überstand die Antikörperausbeute bis zu 75% erhalten wird.

9. Verfahren nach Anspruch 6, wobei der pH der wasserlöslichen Proteinfraktion, erhalten nach der Entfernung der Lipide, auf pH 5,0 eingestellt wird, um die Antikörper weiter auszufällen, um eine Ausbeute von 80-90% zu erhalten.

10. Verfahren nach Anspruch 7, wobei die Ausbeute von Antikörper bis zu dem Ausmaß von 73% beträgt.

11. Verfahren nach Anspruch 1, wobei die hyperimmunen Eier täglich gesammelt und bis zu weiterer Verwendung bei 40°C gelagert werden.

12. Verfahren nach Anspruch 1, wobei die Herstellung des Antikörpers vom 7. Tag nach der Immunisierung begonnen wird und für 60 Tage fortgesetzt wird.

13. Verfahren nach Anspruch 1, wobei der Titer des hergestellten Antikörpers 165-225 mg/ml beträgt.

14. Verfahren nach Anspruch 1, wobei der hergestellte Antikörper aus Eidotter gegenüber den unter Verwendung von Säugetieren hergestellten polyklonalen/monoklonalen Antikörpern mehr/gleich empfindlich ist.

## Revendications

1. Procédé de production d'anticorps de jaune d'oeuf se liant à des pesticides organochlorés à petites molécules, ledit procédé comprenant :
récolter les anticorps du jaune d'oeuf de volailles immunisées, où lesdites volailles ont été obtenues précédemment par les étapes consistant à :
(a) sélectionner des volailles qui conviennent;
(b) immuniser les volailles avec un adjuvant complet connu, chaque ml dudit adjuvant comprenant 1 mg de *Mycobacterium tuberculosis* tué thermiquement et séché (H37Ra, ATCC 25177), 0,85 ml de paraffine et 0,15 ml de monooléate de mannide;
(c) immuniser les volailles avec 1000 µg de conjugué sélectionné parmi l'haptène DDT-OH, l'haptène octachloro cyclique, l'acide 2,4,5-trichlorophenoxyacétique-β-alanine, dans le muscle de la poitrine;
(d) immuniser de nouveau les volailles avec le conjugué haptène-protéine tel que présenté à l'étape (c), avec 500 µg du conjugué haptène désiré;
(e) immuniser les volailles avec le conjugué haptène-protéine à des intervalles de deux, trois et cinq semaines; et
(f) immuniser ensuite les volailles avec le conjugué haptène-protéine à des intervalles de cinq semaines aussi longtemps que la volaille pond des oeufs.

2. Procédé tel que revendiqué dans la revendication 1, dans lequel les conjugués haptène-protéine désirés possèdent des propriétés de liaison au DDT, à l'endosulfane et à l'hexachlorohexane.

3. Procédé tel que revendiqué dans la revendication 1, dans lequel le conjugué haptène-protéine, DDT-OH, se liant au DDT, est utilisé à l'étape (c) de la revendication 1 et est produit comme suit :
(a) succinyler du 2,2-bis(4-chlorophényl)-1,1,1-trichloroéthanol pendant la nuit en utilisant un excédent d'anhydride succinique dans de la pyridine pour obtenir du N-hydroxy-succinimide;
(b) mettre à réagir 183,5 mg de N-hydroxy-succinimide 0,5 mmol, dans du dichlorométhane en présence de dicyclohexylurée et d'un catalyseur à base de diméthylaminopyridine selon le rapport de 1:1:1:1,2 (haptène:NHS:DCC:DMAP) pour le convertir en un ester actif de N-hydroxy-succinimide; et
(c) obtenir l'ester actif d'haptène DDT-OH à utiliser en conjugaison, en isolant la dicyclohexylurée et en évaporant le dichlorométhane.

4. Procédé tel que revendiqué dans la revendication 1, dans lequel le conjugué haptène-protéine, haptène octachloro-cyclique, se liant à l'endosulfane, est utilisé à l'étape (c) de la revendication 1 et est produit comme suit :
(a) dissoudre environ 3,73 g d'heptachlore dans 0,1 mol d'acide acétique glacial 0,1 mol en réchauffant;
(b) dissoudre 1,085 g d'hypochlorite de tert-butyle dans 0,1 mmol d'acide acétique glacial et ajouter à la première solution telle qu'obtenue à l'étape (a);
(c) chauffer le mélange à reflux dans un bain-marie pendant 1 heure;
(d) séparer les fins cristaux de dérivé acétyle-chloré de l'heptachlore;
(e) laver les cristaux avec de l'acétone et sécher à l'air;
(f) obtenir le produit cristallin selon un rendement d'environ 3,02 g, point de fusion 238°C, 1,09 g.
(g) traiter le produit pour obtenir le pré-haptène, 1,3,4,5,6,7,8,8-octachloro-2-hydroxy-4,7-méthano-3a et 4,7,7a-tétrahydroindane;
(h) dissoudre le pré-haptène dans du dichlorométhane en ajoutant du N-hydroxysuccinimide et refroidir le mélange à 0°C;
(i) ajouter du dicyclohexylcarbodiimide suivi par de la diméthylaminopyridine;
(j) mélanger le mélange pendant la nuit; et
(k) retirer le dichlorométhane en filtrant et évaporer le dichlorométhane pour obtenir l'ester actif d'endosulfane.

5. Procédé tel que revendiqué dans la revendication 1, dans lequel le conjugué haptène, acide 2,4,5-trichlorophénoxyacétique-β-alanine (TCB), se liant à l'hexachlorohexane, est utilisé à l'étape (c) de la revendication 1 et est produit comme suit:
(a) ajouter un bras espaceur de β-alanine à de l'acide 2,4,5-trichlorophénoxyacétique en mettant en suspension 2,55 g d'acide 2,4,5-trichlorophénoxyacétique 10 mM, dans 5,95 ml de chlorure de thionyle (9,50 mmol);
(b) chauffer à reflux pendant 1 heure et retirer par évaporation le chlorure de thionyle qui n'a pas réagi;
(c) agiter le produit avec de la β-alanine (9 mmol, 0,66 g dans 7,4 ml de NaOH 1 M) à 0°C;
(d) chauffer le produit pendant 16 heures à température ambiante;
(e) isoler l'acide résultant par acidification;
(f) partager dans de l'acétate d'éthyle;
(g) laver avec de l'eau et de la saumure;
(h) obtenir un rendement d'haptène sous forme de produit brut contenant de l'acide 2,4,5-trichlorophénoxyacétique (2,4,5-T) en tant qu'impureté;
(i) dissoudre l'impureté dans de l'acétone pour obtenir des flocons incolores d'haptène trichlorobenzène (TCB);
(j) filtrer et laver l'haptène TCB incolore avec de l'acétone et sécher à l'air;
(k) en utilisant des plaques d'aluminium pré-revêtues de gel de silice et un mélange de chloroforme et de méthanol selon un rapport de 85:15 comme éluant, une seule tache a été révélée par analyse TLC à Rf-0,45, en vaporisant avec de la o-tolidine 2% dans de l'acétone et en exposant à une lumière UV/lumière du soleil, à une plage de fusion de 169-70°C.
(l) synthétiser l'ester actif de l'haptène 2,4,5,-T-β-alanine à une plage de fusion de 102-104°C par dissolution dans du dichlorométhane;
(m) ajouter du N-hydroxysuccinimide et refroidir le mélange dans un bain glacé;
(n) ajouter du sulfoxyde de diméthyle (DMSO) goutte à goutte au mélange jusqu'à la dissolution de l'haptène;
(o) ajouter du dicyclohexylcarbodiimide au mélange puis ajouter le catalyseur à base de diméthylaminopyridine;
(p) agiter le mélange pendant la nuit et laisser la température monter lentement à la température ambiante;
(q) filtrer et évaporer l'acétone; et
(r) séparer l'ester actif sous forme de solide incolore;

6. Procédé tel que revendiqué dans la revendication 1, dans lequel la récolte des anticorps se fait comme suit :
(a) obtenir le jaune d'oeuf sans casser le jaune;
(b) ajouter 100 ml de solution saline à tampon Tris pour tous les 10 ml de jaune;
(c) retirer le précipité par centrifugation;
(d) ajouter la solution de précipitation consistant en chlorure de magnésium et acide phosphotungstique au surnageant pour centrifugation;
(e) jeter la pastille;
(f) ajouter une fraction protéique soluble dans l'eau, du polyéthylène glycol 12%, au surnageant;
(g) incuber pendant 10 minutes et puis centrifuger de nouveau;
(h) séparer l'anticorps par précipitation;
(i) ajouter 10 ml de tampon phosphate 10 mM pour dissoudre le précipité;
(j) refroidir la solution d'anticorps à 0°C;
(k) ajouter 10 ml d'éthanol pré-refroidi;
(l) centrifuger la solution à 4°C et dissoudre le sédiment dans du tampon phosphate 10 mM; et
(m) dialyser contre un tampon phosphate pendant 24 heures à 4°C pour obtenir le rendement d'anticorps.

7. Procédé tel que revendiqué dans la revendication 1, dans lequel la récolte des anticorps est effectuée comme suit :
(a) obtenir le jaune d'oeuf de la coquille d'oeuf sans briser la membrane du jaune;
(b) ajouter 10 ml d'eau distillée pour tous les 10 ml de jaune;
(c) ajouter environ 0,15% de kappa-carraghénane et laisser à agiter pendant 30 minutes à température ambiante;
(d) filtrer et centrifuger la solution pendant 15 minutes;
(e) passer à travers une colonne DEAE-Sephacel préparée avec du tampon phosphate 20 mM, pH 8,0.
(f) éluer avec du tampon phosphate 0,2 M, pH 8,0;
(g) recueillir l'éluat et relever l'absorption à 280 nm; et
(h) rassembler et conserver à 4°C les fractions pics contenant l'anticorps.

8. Procédé tel que revendiqué dans la revendication 6, dans lequel le lipide du jaune d'oeuf est retiré deux fois par précipitation en utilisant la solution de précipitation à l'acide phosphotungstique et au chlorure de magnésium, et centrifugé pour obtenir du surnageant un rendement d'anticorps de jusqu'à 75%.

9. Procédé tel que revendiqué dans la revendication 6, dans lequel le pH de la fraction protéique soluble dans l'eau obtenue après l'enlèvement des lipides, est ajusté à pH 5,0 afin de retirer d'autres anticorps par précipitation pour obtenir un rendement de 80-90%.

10. Procédé tel que revendiqué dans la revendication 7, dans lequel le rendement d'anticorps est à un degré de 73%.

11. Procédé tel que revendiqué dans la revendication 1, dans lequel les oeufs hyper-immuns sont ramassés tous les jours et conservés à 40°C jusqu'à emploi ultérieur.

12. Procédé tel que revendiqué dans la revendication 1, dans lequel la production de l'anticorps commence au septième jour après l'immunisation et se poursuit pendant 60 jours.

13. Procédé tel que revendiqué dans la revendication 1, dans lequel le titre d'anticorps produit est de 165-225 mg/ml.

14. Procédé tel que revendiqué dans la revendication 1, dans lequel l'anticorps de jaune d'oeuf produit est plus/pareillement sensible aux anticorps polyclonaux/monoclonaux produits en utilisant des mammifères.
